# EUROPÄISCHE PATENTSCHRIFT

(11) **EP 3 076 369 B1**
(45) Veröffentlichungstag und Bekanntmachung des Hinweises auf die Patenterteilung: **29.11.2017**
(21) Anmeldenummer: 16161420.1
(22) Anmeldetag: 28.06.2012
(51) Int. Cl.: G06T 7/33, G06T 11/60

(54) **VERFAHREN UND VORRICHTUNG ZUM DARSTELLEN EINES OBJEKTES**
METHOD AND DEVICE FOR REPRESENTING AN OBJECT
PROCEDE ET DISPOSITIF DESTINES A LA REPRESENTATION D'UN OBJET

(30) Priorität: 28.06.2011 DE 102011078212
(43) Veröffentlichungstag der Anmeldung: 05.10.2016
(62) Teilanmeldung aus: 12745647.3
(73) Patentinhaber: Scopis GmbH, 10179 Berlin (DE)
(72) Erfinder: KOSMECKI, Bartosz, 10965 Berlin (DE); REUTTER, Andreas, 10437 Berlin (DE); ÖZBEK, Christopher, 10823 Berlin (DE)
(74) Vertreter: Tegethoff, Sebastian

(56) Entgegenhaltungen:
- WO-A2-2011/063266
- JOSHUA LEVEN ET AL: "DaVinci Canvas: A Telerobotic Surgical System with Integrated, Robot-Assisted, Laparoscopic Ultrasound Capability", 1 January 2005 (2005-01-01), MEDICAL IMAGE COMPUTING AND COMPUTER-ASSISTED INTERVENTION - MIC CAI 2005 LECTURE NOTES IN COMPUTER SCIENCE;;LNCS, SPRINGER, BERLIN, DE, PAGE(S) 811 - 818, XP019021602, ISBN: 978-3-540-29327-9 * abstract * * figures 1-3 * * Abschnitt 1,3 *
- HAYASHIBE M ET AL: "Laser-pointing endoscope system for intra-operative 3D geometric registration", PROCEEDINGS OF THE 2001 IEEE INTERNATIONAL CONFERENCE ON ROBOTICS AND AUTOMATION. ICRA 2001. SEOUL, KOREA, MAY 21 - 26, 2001; [PROCEEDINGS OF THE IEEE INTERNATIONAL CONFERENCE ON ROBOTICS AND AUTOMATION], NEW YORK, NY : IEEE, US, vol. 2, 21 May 2001 (2001-05-21), pages 1543-1548, XP010550367, DOI: 10.1109/ROBOT.2001.932830 ISBN: 978-0-7803-6576-6
- CHRISTOPH STAUB ET AL: "Automation of tissue piercing using circular needles and vision guidance for computer aided laparoscopic surgery", 2010 IEEE INTERNATIONAL CONFERENCE ON ROBOTICS AND AUTOMATION : ICRA 2010 ; ANCHORAGE, ALASKA, USA, 3 - 8 MAY 2010, IEEE, PISCATAWAY, NJ, USA, 3 May 2010 (2010-05-03), pages 4585-4590, XP031743536, ISBN: 978-1-4244-5038-1

## Beschreibung

Die Erfindung betrifft ein Verfahren zum Darstellen eines Objektes, insbesondere eines biologischen Gewebes, gemäß Anspruch 1 sowie eine Vorrichtung zum Darstellen eines Objektes gemäß Anspruch 14. Aus der Medizintechnik sind Ultraschall- und Endoskopievorrichtungen bekannt, die einen operativen Eingriff unter Ultraschallkontrolle bzw. Sichtkontrolle erlauben. Beispielsweise offenbart die US 601 97 24 A eine entsprechende Ultraschallvorrichtung.

Die Publikation von Leven et al (Med Image Comput Comput Assist Interv. 2005, 8 (Pt 1), Seiten 811-818) beschreibt ein telerobotisches chirurgisches System (DaVinci Canvas) mit integrierter Roboter-unterstützter laparoskopischer Ultraschallfähigkeit. Das in diesem Dokument beschriebene System besteht aus der Integration einer starren laparoskopischen Ultraschallsonde, Video-Tracking von Ultraschall-Sondenbewegungen, Endoskop- und Ultraschall-Kalibrierung und Registrierung, autonomen Roboterbewegungen und der Anzeige von registrierten 2D- und 3D-Ultraschallbildern.

Das der Erfindung zugrunde liegende Problem besteht darin, ein Verfahren anzugeben, das eine möglichst gute Kontrolle eines an einem Objekt erfolgenden Eingriffs ermöglicht. Des Weiteren soll eine entsprechende Vorrichtung bereitgestellt werden.

Diese Probleme werden durch das Verfahren mit den Merkmalen gemäß Anspruch 1 sowie durch die Vorrichtung mit den Merkmalen gemäß Anspruch 14 gelöst. Weiterbildungen der Erfindung sind in den abhängigen Ansprüchen angegeben.

Danach wird ein Auswahlverfahren zur Selektion von Bildpunkten bei der Darstellung eines Objektes bei intraoperativer Bildgebung zur Verfügung gestellt, , insbesondere eines biologischen Gewebes, mit den Schritten:
a) Erzeugen eines ersten Bildes zumindest eines Teilbereichs des Objekts mit Hilfe einer Endoskopievorrichtung als erster Vorrichtung;
b) Erzeugen eines zweiten Bildes zumindest des Teilbereichs des Objekts aus Schritt a) mit Hilfe einer zweiten Vorrichtung;
c) Ermitteln von ersten Koordinaten zumindest einiger Bildpunkte des zweiten Bildes in einem ersten Koordinatensystem;
d) Ermitteln von zweiten Koordinaten der Bildpunkte des zweiten Bildes durch Abbilden der ersten Koordinaten in ein zweites Koordinatensystem, das von dem ersten Koordinatensystem verschieden und der ersten Vorrichtung zugeordnet ist; und
e) Erzeugen eines kombinierten Bildes des Objekts aus dem ersten Bild und für ausgewählte Bildpunkte aus dem zweiten Bild unter Verwendung der ermittelten zweiten Koordinaten der Bildpunkte des zweiten Bildes, dadurch gekennzeichnet, dass
f) die Selektion der zu kombinierenden Bildpunkte bestimmt wird durch die Auswahl eines Teilbereiches des Objektes, wobei
   - die erste Vorrichtung eine Lichtquelle aufweist und der auszuwählende Teilbereich des Objektes durch Erzeugen mindestens eines Lichtflecks auf dem Objekt mit Hilfe der Lichtquelle markiert wird, oder
   - der Teilbereich des Objekts mit Hilfe eines separaten, navigierten Instruments markiert wird,
g) so dass das Erzeugen des kombinierten Bildes bei der intraoperativen Bildgebung durch Ersetzen oder Überlagern von Bildpunkten des ersten Bildes innerhalb des ausgewählten Teilbereichs des Objektes durch die ausgewählten Bildpunkte des zweiten Bildes erfolgt.

Die erste und die zweite Vorrichtung sind insbesondere zur intraoperativen Bildgebung ausgebildet. Das erste und das zweite Bild, das mit der ersten bzw. der zweiten Vorrichtung erzeugt wird, entsteht z.B. durch Abbildung (Projektion) zumindest eines Teilbereichs des Objekts in eine Bildebene oder durch eine 3D-Darstellung des Objektes. Beispielsweise ist das erste Bild ein Endoskopiebild oder ein Stereoendoskopiebild und die erste Vorrichtung entsprechend eine Endoskopievorrichtung. Denkbar ist natürlich auch, dass es sich bei der ersten Vorrichtung um eine nicht endoskopische Vorrichtung handelt.

Das zweite Bild ist z.B. ein Ultraschallbild, ein MRT-Bild, ein Röntgenbild, ein nuklearradiologisches Bild, ein MRT-Bild und/oder ein Einzelphotonen-Emissions-Tomografie-Bild und die zweite Vorrichtung entsprechend eine Ultraschallvorrichtung, eine MRT-vorrichtung, eine Röntgenvorrichtung, eine nuklear-radiologische Vorrichtung, eine MRT-Vorrichtung und/oder eine Einzelphotonen-Emissions-Tomografie-Vorrichtung (Single-photon emission computed tomography (SPECT)-Vorrichtung).

Das Ermitteln der zweiten Koordinaten der Bildpunkte des zweiten Bildes im zweiten Koordinatensystem ermöglicht insbesondere eine perspektivisch korrekte (lagerichtige) Integration zumindest eines Teils des zweiten Bildes in das erste Bild.

Beispielsweise weist die erste Vorrichtung ein optisches System (z.B. eine Kamera) auf, wobei z.B. das zweite Koordinatensystem einer Bildebene des optischen Systems zugeordnet ist. Das optische System der ersten Vorrichtung ist beispielsweise eine Videokamera (z. B. eine CCD-Kamera), mit der ein Echtzeitbild des zu untersuchenden Objektes erzeugt werden kann. Analog kann auch mittels der zweiten Vorrichtung ein Echtzeitbild erzeugt werden, so dass ein kombiniertes Echtzeitbild des Objektes realisiert werden kann.

Bei dem darzustellenden Objekt handelt es sich insbesondere um ein biologisches Gewebe (z.B. ein Weichgewebe), wobei das erfindungsgemäße Verfahren zur Kontrolle einer Operation des Gewebes (z. B. einem Entfernen eines Tumors, etwa eines Myoms) verwendet werden kann, d. h. intraoperativ. Es ist jedoch auch denkbar, dass das erfindungsgemäße Verfahren in nichtmedizinischen Anwendungen eingesetzt wird, z. B. zur Kontrolle einer Reparatur an einem Bauteil.

Durch die perspektivisch korrekte Kombination der Bilddaten der ersten und der zweiten Vorrichtung (z.B. von Endoskopie- und Ultraschallbilddaten) wird z.B. einem Operateur zusätzlich zu dem Endoskopiebild eine Tiefendarstellung des relevanten Operationsgebietes zur Verfügung gestellt. Beispielsweise kann der Operateur mit Hilfe des kombinierten Bildes eine Tiefenausdehnung eines Tumors beurteilen, die im reinen Endoskopiebild nicht zu erkennen ist. Durch die zusätzliche Tiefeninformation ist der Operateur z.B. in der Lage, seine Planung der Operation oder den Befund im Verlauf der Operation zu aktualisieren.

Bei dem zweiten Koordinatensystem, in das die Koordinaten der Bildpunkte des zweiten Bildes abgebildet werden, handelt es sich beispielsweise um ein zweidimensionales Koordinatensystem, das z.B. einer Bildebene einer Kamera der ersten Vorrichtung zugeordnet ist. In der Bildebene der Kamera befindet sich z. B., wie oben bereits angesprochen, ein CCD-Chip, wobei sich z. B. der Ursprung des zweiten Koordinatensystems in einer Ebene befindet, die einer Ebene entspricht, entlang derer sich der CCD-Chip erstreckt, d. h. die Koordinatenachsen des zweiten Koordinatensystems verlaufen in einer Ebene, deren Lage der Bildebene der Kamera der ersten Vorrichtung entspricht. Denkbar ist auch, dass es bei der ersten Vorrichtung um eine Stereoendoskopievorrichtung handelt, die mittels zweier optischer Systeme (Kameras) zwei perspektivisch unterschiedliche Bilder erzeugt. In diesem Fall erfolgt eine Abbildung der ersten Koordinaten des zweiten Bildes sowohl in das (zweite) Koordinatensystem, das der Bildebene der ersten Kamera zugeordnet ist, als auch in das (weitere zweite) Koordinatensystem, das der Bildebene der zweiten Kamera zugeordnet ist, um das zweite Bild lagerichtig in beide Bilder der Stereoendoskopievorrichtung lagerichtig integrieren zu können.

Das Abbilden der ersten Koordinaten in das zweite Koordinatensystem erfolgt insbesondere mittels einer Abbildung (insbesondere mittels einer Transformation), die durch Kalibrieren der ersten Vorrichtung (z.B. in Form einer Endoskopievorrichtung) bestimmt wird. Dieser Abbildung liegt insbesondere ein Modell der ersten Vorrichtung (insbesondere eines optischen Systems der ersten Vorrichtung) zugrunde, wobei durch die Kalibrierung Parameter des optischen Systems bestimmt werden. Zur Kalibrierung wird beispielsweise ein Kalibriermuster mit der ersten Vorrichtung abgebildet, wobei die räumlichen Koordinaten des Kalibriermusters (z.B. relativ zu der ersten Vorrichtung) mit Hilfe einer Positionsbestimmungsvorrichtung bestimmt und den Koordinaten des Bildes des Kalibriermusters in der Bildebene der ersten Vorrichtung zugeordnet werden. Ein geeignetes Kalibrierverfahren, insbesondere für eine erste Vorrichtung in Form einer Endoskopievorrichtung, das auch zur Kalibrierung einer Abstandsbestimmungsvorrichtung einer Endoskopievorrichtung verwendet werden kann, ist in der deutschen Patentanmeldung DE 10 2010 042 540.0 vom 15.10.2010 beschrieben, auf die insofern ausdrücklich Bezug genommen wird.

Durch das Kalibrieren der ersten Vorrichtung wird auch eine Verzerrung des Objektes durch eine Optik der ersten Vorrichtung erfasst, d. h., es werden z.B. auch Verzerrungsparameter des erwähnten Modells des optischen Systems der ersten Vorrichtung ermittelt. Insbesondere erfolgt die Abbildung der ersten Koordinaten der Bildpunkte des zweiten Bildes in das zweite Koordinatensystem unter Verwendung dieser Verzerrparameter, d. h. einer Verzerrfunktion.

Beispielsweise erfolgt sowohl das Kalibrieren der ersten Vorrichtung als auch ein Kalibrieren der zweiten Vorrichtung unter Verwendung jeweils eines Kalibrierkörpers, wobei die Kalibrierkörper (z.B. einstückig) miteinander verbunden sind (d.h. eine Baugruppe bilden), wobei die räumliche Position der Kalibrierkörper insbesondere über eine Positionsbestimmungsvorrichtung bestimmbar ist.

Gemäß einer weiteren Ausgestaltung der Erfindung umfasst das Ermitteln der zweiten Koordinaten der Bildpunkte des zweiten Bildes ein Bestimmen der räumlichen Position der zweiten Vorrichtung (z.B. eines Ultraschallkopfes einer Ultraschallvorrichtung) mit Hilfe einer Positionsbestimmungsvorrichtung und ein Abbilden der ersten Koordinaten der Bildpunkte in räumliche Koordinaten in einem der Positionsbestimmungsvorrichtung zugeordneten räumlichen Koordinatensystem ("Weltkoordinatensystem"). Bei der Positionsbestimmungsvorrichtung handelt es sich beispielsweise um ein klinisches Navigationssystem oder einen Bestandteil eines klinischen Navigationssystems, das unter Verwendung von Markierungselementen (die insbesondere an der zweiten Vorrichtung, z.B. einem Ultraschallkopf, angeordnet sind) und eines Messgeräts (wie insbesondere eine Messkamera oder ein Magnetfeldsensor) eine Bestimmung der räumlichen Position (d. h. der Position und der Ausrichtung) eines Gegenstandes wie dem Ultraschallkopf ermöglicht. Die Zuordnung der Bilddaten der zweiten Vorrichtung zu räumlichen Koordinaten (die den räumlichen Koordinaten von Punkten des im zweiten Bild dargestellten Objektes entsprechen) erfolgt insbesondere nach einer entsprechenden Kalibrierung der zweiten Vorrichtung.

Als Markierungselemente können aktive (selbst leuchtende) Elemente, wie z.B. LEDs, passive (nicht selbstleuchtende) Elemente, wie z.B. reflektierende Kugeln, Folien, spezielle Muster (flache Zielmarken, Lasergravuren oder natürliche Muster wie Ecken und Kanten) oder Magnetfeldquellen (z.B. in Form von stromdurchflossenen Spulen), verwendet werden. Zur Positionsbestimmung kann auch die Anbringung von Messgerät und Markierungselement vertauscht sein, d.h. das Messgerät an der jeweiligen Vorrichtung befestigt sein und die Markierungselemente im Raum positioniert sein, wie. es z.B. im Fall eines mobilen Endgeräts der Fall wäre, welches im Raum fest platzierte Markierungselemente optisch detektiert oder im Falle eines Magnetfeldsensors, der ein in den Raum ausgestrahltes Magnetfeld mit Markierungsfunktion detektiert.

Eine Messkamera einer Positionsbestimmungsvorrichtung basiert insbesondere auf dem Prinzip einer Stereokamera, d.h. sie weist beispielsweise zwei zueinander beabstandete Sensorelemente (insbesondere CCD-Chips) auf, die von einem Gegenstand (insbesondere von einem Markierungselement der Positionsbestimmungsvorrichtung) Licht unter unterschiedlichen Winkeln empfangen, so dass die räumliche Position des Objektes aus den Daten der Sensorelemente rekonstruiert werden kann.

Somit können die Raumpositionen der Markierungselemente, d.h. ihre Raumkoordinaten in einem vorgegebenen, der Positionsbestimmungsvorrichtung zugeordneten Koordinatensystem, bestimmt werden. Ist die relative Position der Markierungselemente in Bezug zu dem System, an dem sie angebracht sind, bekannt, kann aus den Raumpositionen der Markierungselemente auf die Position des Systems geschlossen werden. Derartige Positionsbestimmungsvorrichtungen sind an sich jedoch bekannt, so dass hierauf nicht weiter eingegangen werden soll.

Analog kann mit einer Positionsbestimmungsvorrichtung, die z. B. auch zur Bestimmung der räumlichen Position der zweiten Vorrichtung (z.B. des Ultraschallkopfes einer Ultraschallvorrichtung) dient, die räumliche Position der ersten Vorrichtung (z.B. in Form einer Endoskopievorrichtung und insbesondere deren Endoskopschaftes) bestimmt werden, wobei das Ermitteln der zweiten Koordinaten der Bildpunkte des ersten Bildes unter Verwendung der bestimmten räumlichen Position der ersten Vorrichtung erfolgt.

In einem weiteren Ausführungsbeispiel der Erfindung umfasst das Erzeugen des kombinierten Bildes ein Ermitteln von Koordinaten zumindest einiger der Bildpunkte des ersten Bildes in dem zweiten Koordinatensystem, wobei die Bildpunkte des ersten Bildes durch Bildpunkte des zweiten Bildes mit den entsprechenden zweiten Koordinaten ersetzt oder überlagert werden. Ein Überlagern (Überblenden) der Bilder kann z. B. dadurch erzeugt werden, dass die Intensitäten der Bildpunkte des ersten Bildes und des zweiten Bildes addiert werden. Denkbar ist auch, dass ein Benutzer, den Überlagerungsgrad (Überblendgrad) festlegen kann. Selbstverständlich kann das erste Bild und/oder das zweite Bild vor der Kombination bearbeitet werden, z. B. unter Verwendung eines Filters oder mehrerer Filter (z.B. zum Einführen eines Deckkraftfaktors).

Das zweite Bild kann auch in Form eines 3D-Bildes (z.B. eines 3D-Ultraschallbildes, z.B. einer 3-D-Rekonstruktion) vorliegen, das durch Erfassen (durch Bewegen z.B. des Ultraschallkopfes einer Ultraschallvorrichtung) einer Mehrzahl von Bildebenen erzeugt werden kann. Entsprechend dem oben beschriebenen Verfahren werden den räumlichen Koordinaten des zweiten Bildes (zweiten) Koordinaten der Bildebene der ersten Vorrichtung zugeordnet. In dieser Variante der Erfindung wird somit eine 3D-Ansicht des Objektes in das erste Bild integriert. Möglich ist auch, dass die zur Realisierung des erfindungsgemäßen Verfahrens verwendete Vorrichtung so ausgebildet ist, dass ein Benutzer zwischen der 3D-Einblendung und der Einblendung des aktuell von der zweiten Vorrichtung erzeugten 2D-Bildes umschalten oder die Integration des zweiten Bildes auch komplett abschalten kann.

Möglich ist auch, dass beim Erzeugen des kombinierten Bildes eine Teilauswahl von Bildpunkten des ersten Bildes durch ein Auswahlverfahren bestimmt wird, und die bestimmten Bildpunkte durch Bildpunkte des zweiten Bildes mit den entsprechenden zweiten Koordinaten ersetzt oder überlagert werden. Beispielsweise wird zur Auswahl von zu ersetzenden Bildpunkten ein Teilbereich im ersten Bild markiert.

Denkbar ist darüber hinaus auch, dass (z.B. in dem ersten Bild) ein Teilbereich (Teilabschnitt) des Objektes ausgewählt (z.B. markiert) wird und das Erzeugen des kombinierten Bildes durch Ersetzen oder Überlagern von Bildpunkten des ersten Bildes innerhalb des Teilbereiches des Objektes erfolgt. Insbesondere erfolgt die Kombination des ersten und des zweiten Bildes (das Ersetzen und/oder Überlagern der Bildpunkte) ausschließlich in dem markierten Teilbereich. Insbesondere wird als Teilbereich ein Teilabschnitt des mit dem ersten Bild dargestellten Teilbereich des Objektes ausgewählt bzw. es wird ein Teilbereich des ersten Bildes ermittelt, der dem ausgewählten Teilbereich (Teilabschnitt) des Objektes entspricht. Denkbar ist, dass der Teilbereich des Objektes dadurch ausgewählt wird, dass, wie oben erwähnt, ein Teilbereich des ersten Bildes ausgewählt, z.B. im ersten Bild markiert, wird.

Das Markieren (Segmentieren) des Teilbereiches kann z. B. dazu dienen, eine zu operierende Gewebestruktur (z. B. einen Tumor) zu kennzeichnen, so dass die für den Operateur relevante Information des zweiten Bildes hauptsächlich oder ausschließlich in diesem Teilbereich eingeblendet wird, um einerseits die gewünschte Tiefeninformation bereitzustellen, den Operateur jedoch nicht mit unnötigen Bilddaten zu belasten. Denkbar ist auch, dass der markierte Teilbereich einer Bewegung des Objektes folgt. Dies wird insbesondere dadurch ermöglicht, dass auch an dem Objekt (z.B. einem zu operierenden Patienten) Markierungselemente angeordnet werden können, deren Position mittels der erwähnten Positionsbestimmungsvorrichtung verfolgt wird, so dass die Objektposition und somit bei der Erzeugung des kombinierten Bildes berücksichtigt werden kann. Alternativ kann dies auch ohne Markierungselemente über eine Erkennung von gleichen Merkmalen in nacheinander folgenden Bildern und damit z.B. über eine Verfolgung einer Deformation des Objektes erreicht werden ("nichtrigide Bildregistrierung"); s.u..

Denkbar ist, dass der Teilbereich des Objektes anhand einer Rekonstruktion eines dreidimensionalen Bereichs des Objektes ausgewählt wird, wobei die Rekonstruktion unter Verwendung einer Mehrzahl von mit der zweiten Vorrichtung erzeugten Bildern generiert wird. Beispielsweise umfasst die Rekonstruktion des dreidimensionalen Bereichs des Objektes eine Interpolation zwischen zwei zeitlich aufeinanderfolgenden Bildern der zweiten Vorrichtung in Abhängigkeit von ihrer jeweiligen, mittels der Positionsbestimmungsvorrichtung ermittelten räumlichen Positionen umfasst.

Die Interpolation der zwei zeitlich aufeinanderfolgenden Bilder der zweiten Vorrichtung und/oder eine Speicherung von durch die Interpolation erzeugten Daten kann auf einer Grafikkarte erfolgen, wobei die Interpolation auf der Grafikkarte z.B. parallelisiert berechnet wird.

Gemäß einer Weiterbildung der Erfindung erfolgt das Markieren des Objektes mittels einer Lichtquelle (insbesondere eines Lasers) der ersten Vorrichtung, wobei der Teilbereich dadurch markiert wird, dass mit der Lichtquelle zumindest ein Lichtfleck auf dem Objekt erzeugt wird. Insbesondere wird eine Mehrzahl von Lichtflecken (Stützpunkten) erzeugt, wobei die Lichtflecke (Lichtpunkte) gleichzeitig oder nacheinander erzeugt werden können. Falls die Lichtflecken nacheinander erzeugt werden, wird z.B. für jeden Lichtfleck ein den Lichtfleck zeigendes erstes Bild gespeichert, wobei während des Markierungsvorgangs (insbesondere in Echtzeit) oder nach Beenden des Markierungsvorgangs die entsprechenden ersten Bilder ausgewertet werden und der sich aus den nacheinander erzeugten Lichtflecken zusammensetzende Teilbereich in Form z.B. einer 2D-Polygonfläche oder eines Volumens bestimmt wird.

Gemäß einer Weiterbildung der Erfindung werden zu einem ersten Zeitpunkt Ausgangskoordinaten (insbesondere im Weltkoordinatensystem) des Teilbereichs und zu einem zweiten Zeitpunkt aktualisierte Koordinaten des Teilbereichs in Abhängigkeit von der aktuellen Position und/oder Form des Objektes bestimmt. Mit anderen Worten erfolgt eine Verfolgung der Position und/oder der Form des Objekts, wobei das Objekt z.B. relativ zum Weltkoordinatensystem und damit zum Koordinatensystem der ersten Vorrichtung z.B. mittels Markierungselementen, die relativ zum Objekt angeordnet werden und über rigide und nichtrigide Transformationen zu diesem in Beziehung stehen.

Denkbar ist auch, dass das Markieren dadurch erfolgt, dass ein von der Lichtquelle erzeugter Lichtstrahl kontinuierlich über das Objekt geführt wird und eine mit der ersten Vorrichtung aufgenommene Sequenz von Aufnahmen ausgewertet wird.

Zum Markieren des interessierenden räumlichen Teilbereichs des Objektes kann auch eine Abstandsbestimmungsvorrichtung der ersten Vorrichtung verwendet werden. Genauer werden die folgenden Schritte durchgeführt:
- Markieren eines räumlichen Teilbereichs des Objektes mittels einer Mehrzahl von Lichtflecken;
- Ermitteln der räumlichen Position der ersten Vorrichtung unter Verwendung einer Positionsbestimmungsvorrichtung;
- Ermitteln des Abstandes zwischen der ersten Vorrichtung und den erzeugten Lichtflecken mit Hilfe einer Abstandsbestimmungsvorrichtung der ersten Vorrichtung;
- Ermitteln der räumlichen Position des Teilbereichs durch Bestimmen der räumlichen Koordinaten der Lichtflecke im Koordinatensystem der Positionsbestimmungsvorrichtung unter Verwendung der ermittelten räumlichen Position der ersten Vorrichtung und des ermittelten Abstandes.

Die Positionsbestimmungsvorrichtung ist insbesondere ein klinisches Navigationssystem, wie oben bereits erläutert. Die Abstandsbestimmungsvorrichtung, mit der sich insbesondere ein Abstand zwischen einem dem abzubildenden Objekt zuzuwendenden Abschnitt der ersten Vorrichtung und dem Objekt bestimmen lässt, weist z.B. Mittel (insbesondere in Form eines Lasers) zum Projizieren der Lichtstruktur auf das abzubildende Objekt auf und wirkt z.B. mit einer Abbildungsoptik und einer Kamera der ersten Vorrichtung zusammen.

Die Mittel zum Projizieren sind insbesondere so in Bezug auf eine Abbildungsoptik der ersten Vorrichtung, die zum Abbilden des Objektes in die Bildebene einer Kamera der ersten Vorrichtung dient, angeordnet, dass zwischen dem durch die Mittel zum Projizieren ausgesandten Lichtbündel und einem am Objekt reflektierten Lichtbündel, das von dem Objekt in eine Abbildungsoptik der ersten Vorrichtung fällt, ein Winkel (z.B. 30°) oder ein Abstand zwischen der optischen Achse des Lichtbündels und optischen Achse der Abbildungsoptik besteht, so dass die Position des Bildes des auf dem Objekt erzeugten Lichtmusters in der Bildebene der Kamera von dem Abstand des Objektes zu der ersten Vorrichtung abhängt. Insbesondere ist auch denkbar, dass mit Hilfe der Abstandsbestimmungsvorrichtung ein Ortsvektor zwischen einem Abschnitt der der ersten Vorrichtung und einem Abschnitt der von der Abstandsbestimmungsvorrichtung projizierten Lichtstruktur bestimmt wird, d.h. es wird nicht nur der Abstand, sondern auch die Orientierung einer Verbindungslinie zwischen diesen Abschnitten ermittelt.

Eine geeignete erste Vorrichtung (in Form einer Endoskopievorrichtung), die eine derartige Abstandsbestimmungsvorrichtung aufweist, ist in der bereits oben erwähnten deutschen Patentanmeldung DE 10 2010 042 540.0 beschrieben, auf die auch insofern ausdrücklich Bezug genommen wird.

Es ist jedoch nicht zwingend, dass der interessierende Teilbereich des Objektes unter Verwendung einer Lichtquelle markiert wird. Denkbar ist vielmehr z.B. auch, dass der Teilbereich in dem (in digitaler Form vorliegenden) ersten Bild eingezeichnet wird (z.B. unter Verwendung einer Computermaus oder eines Zeichenstiftes) oder dass der Teilbereich mit Hilfe einer separaten Positionsbestimmungsvorrichtung (z.B. eines navigierten Instruments) bestimmt wird.

Möglich ist, dass der Teilbereich unter Verwendung eines Ray-Casting-Verfahrens in dem ersten und/oder zweiten Bild dargestellt wird, um einen Tiefeneindruck zu vermitteln. Das Ray-Casting-Verfahren ist z.B. eine Maximum-Intensity-Projektion, eine Average-Intensity-Projektion, eine frei definierbare Transferfunktion und/oder eine Oberflächen-Projektion verwendet.

Möglich ist auch, dass die Abstandsbestimmungsvorrichtung mit der ersten Vorrichtung verbunden ist (z.B. ein Bestandteil der ersten Vorrichtung ist) oder dass die Abstandsbestimmungsvorrichtung eine von der ersten Vorrichtung separate Vorrichtung (ein separates Instrument) ist, d.h. insbesondere beabstandet zu der ersten Vorrichtung angeordnet ist.

Des Weiteren ist möglich, dass die Auswahl des interessierenden Teilbereichs algorithmisch erfolgt, wobei die Auswahl z.B. durch Anwenden einer booleschen Prädikatfunktion (z.B. einer Schwellwertfunktion) auf Bildpunkte des zweiten Bildes und/oder eines Bildanalyseverfahrens (z.B. eines Konturerkennungsverfahrens) auf das zweite Bild ermittelt wird. So kann der Teilbereich etwa durch Vorgeben eines Schwellwertes und Auswählen einer Mehrzahl von Bildpunkten des zweiten Bildes (z.B. in Form eines Ultraschallbildes) in Abhängigkeit von dem Schwellwert bestimmt werden. Beispielsweise bezieht sich der vorgegebene Schwellwert auf die Intensität der Bildpunkte, wobei z.B. Bereiche des Objektes ausgewählt werden, denen nur Bildpunkte des zweiten Bildes zugeordnet sind, deren Intensität den Schwellwert übersteigt (unterschreitet). Dieses automatische Auswahl könnte z.B. genutzt werden, um einen zu entfernenden Tumor auszuwählen, wobei zum Auswählen des Tumors dieser lediglich z.B. mit Ultraschall überstrichen werden müsste, um eine entsprechende 3D-Rekonstruktion des Tumors zu erhalten. Hierbei könnte eine Lichtquelle (insbesondere ein Laser) der ersten Vorrichtung dazu dienen, einen Startpunkt für die 3D-Rekonstruktion zu setzen.

Die schwellwertabhängige Auswahl kann insbesondere in Form eines Filters realisiert sein, der den Hintergrund des zweiten Bildes anhand des Schwellenwerts maskiert. Diese Art der Auswahl (Segmentierung) eines Teilbereiches des zweiten Bildes (d.h. des darzustellenden Objektes) kann insbesondere dazu verwendet werden, einen dunklen Hintergrund des zweiten Bildes auszublenden, der bei der Kombination mit dem ersten Bild möglicherweise relevante Bildinhalte überdecken würde. Beispielsweise kann der Schwellwert auch durch einen Benutzer anpassbar sein.

Denkbar ist auch, dass der markierte räumliche Teilbereich des Objektes zu einem geschlossenen Teilbereich extrapoliert wird und ein kombiniertes Bild nur erzeugt wird, wenn der mit der zweiten Vorrichtung erfasste Bereich des Objektes den geschlossenen Teilbereich schneidet. Beispielsweise wird der markierte Teilbereich zu einem geschlossenen Zylinder extrapoliert. Möglich ist auch, dass der geschlossene Teilbereich mit Hilfe der oben beschriebenen 3D-Rekonstruktion (z.B. eines Tumors) ermittelt wird.

Gemäß einer anderen Ausgestaltung der Erfindung wird in das erste Bild (z.B. in Form eines Endoskopiebildes) eine Information bezüglich der räumlichen Position der zweiten Vorrichtung (z.B. des Ultraschallkopfes einer Ultraschallvorrichtung) eingeblendet, um z. B. einem Operateur eine Hilfestellung zur Ausrichtung der zweiten Vorrichtung zu geben ("Augmented Reality" - erweiterte Realität). Beispielsweise helfen die eingeblendeten Informationen dem Operateur, einen markierten, interessierenden Teilbereich des Objektes mit der zweiten Vorrichtung möglichst vollständig zu erfassen. Alternativ oder zusätzlich ist es auch möglich, dass das zweiten Bild (z.B. ein Ultraschallbild) auf einer separaten Anzeige dargestellt wird und in diese Darstellung eine Information bezüglich der Position der ersten Vorrichtung eingeblendet wird. Denkbar ist auch, dass weitere Information eingeblendet werden, z.B. bezüglich eines Laserstrahls einer Abstandsbestimmungsvorrichtung der ersten Vorrichtung, bezüglich eines navigierten Instrumentes, d.h. eines Instrumentes, dessen Position im Weltkoordinatensystem bestimmt wurde, und/oder präoperative Bilddaten.

Darüber hinaus kann die räumliche Lage mindestens eines Werkzeugs mittels der Positionsbestimmungsvorrichtung bestimmt und das Werkzeug unter Berücksichtigung seiner räumlichen Lage und der räumlichen Lage der zweiten Vorrichtung in das zweite Bild eingeblendet werden. Insbesondere wird das zweite Bild mit dem eingeblendeten Werkzeug gemäß Schritt e) mit dem ersten Bild kombiniert.

Möglich ist zudem, dass eine zeitliche Verzögerung der Bildauslösung der ersten Vorrichtung, der zweiten Vorrichtung und der Positionsbestimmung der Positionsbestimmungseinheit ermittelt wird und für die zeitliche Abstimmung durch eine zentrale Verarbeitungseinheit zur Darstellung des kombinierten Bildes zur Verfügung steht.

Die Erfindung betrifft auch eine Vorrichtung zur Darstellung eines Objektes, insbesondere zum Durchführen eines wie oben beschriebenen Verfahrens, mit:
- einer ersten Vorrichtung zum Erzeugen eines ersten Bildes zumindest eines Teilbereichs des Objekts;
- einer zweiten Vorrichtung zum Erzeugen zumindest eines Teilbereichs eines zweiten Bildes des Objekts;
- einer ersten Koordinatenermittlungseinrichtung zum Ermitteln von ersten Koordinaten zumindest einiger Bildpunkte des zweiten Bildes in einem ersten Koordinatensystem;
- einer zweiten Koordinatenermittlungseinrichtung zum Ermitteln von zweiten Koordinaten der Bildpunkte des zweiten Bildes durch Abbilden der ersten Koordinaten in ein zweites Koordinatensystem, das von dem ersten Koordinatensystem verschieden und der zweiten Vorrichtung zugeordnet ist; und
- einer Bilderzeugungseinrichtung zum Erzeugen eines kombinierten Bildes des Objekts aus dem ersten und dem zweiten Bild unter Verwendung der ermittelten zweiten Koordinaten der Bildpunkte des zweiten Bildes.

Die erste und die zweite Koordinatenermittlungseinrichtung sind insbesondere in Form einer Software oder einer entsprechend programmierten elektronischen Einrichtung (Computer, Mikrochip, usw.) ausgebildet. Ähnlich kann auch die Bilderzeugungseinrichtung in Form einer Software.oder einer entsprechend programmierter Hardware (z.B. einer Grafikkarte) ausgebildet sein.

Die Vorrichtung umfasst insbesondere auch Markierungselemente, die an der ersten Vorrichtung (z.B. einer Endoskopievorrichtung) und/oder der zweiten Vorrichtung (z.B. einem Ultraschallkopf einer Ultraschallvorrichtung) angeordnet sind, wobei mittels einer Positionsbestimmungsvorrichtung, die eine räumliche Position der Markierungselemente und somit der ersten Vorrichtung und/oder der zweiten Vorrichtung bestimmt werden kann. 10 Wie oben bereits erwähnt, handelt es sich bei der Positionsbestimmungsvorrichtung insbesondere um ein klinisches Navigationssystem, das eine Positionsbestimmungsvorrichtung wie z.B.eine Messkamera umfasst.

Die Erfindung wird im Folgenden anhand von Ausführungsbeispielen unter Bezugnahme auf die Figuren näher erläutert. Es zeigen:
- Figur 1: eine schematische Ansicht einer Vorrichtung gemäß einem Ausführungsbeispiel der Erfindung;
- Figur 2: ein mit der Vorrichtung aus Figur 1 erzeugtes Endoskopiebild eines Gewebes;
- Figur 3: ein aus einem Endoskopie- und Ultraschallbild erzeugtes kombiniertes Bild des Gewebes; und
- Figuren 4 und 5: weitere Beispiele eines mit dem erfindungsgemäßen Verfahren erzeugten kombinierten Bildes.

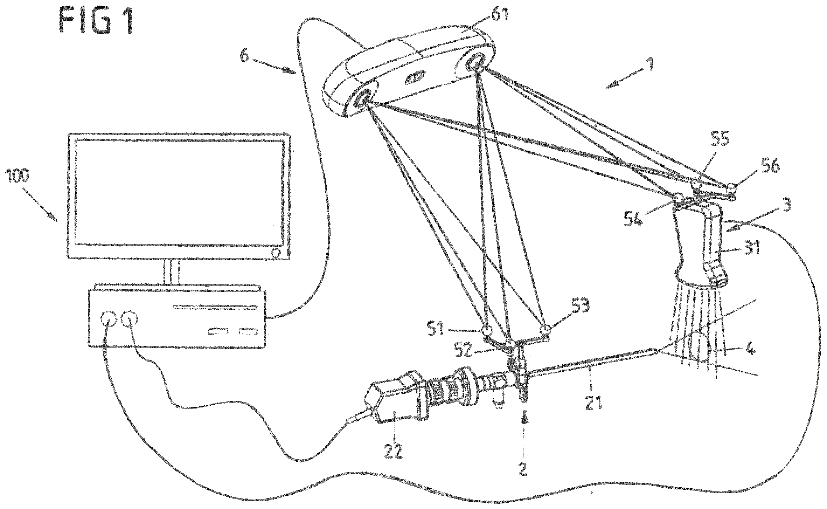

Die in Figur 1 gezeigte erfindungsgemäße Vorrichtung 1 umfasst eine erste Vorrichtung in Form einer Endoskopievorrichtung 2 (z.B. in Form einer Hystoroskopievorrichtung) sowie eine zweite Vorrichtung in Form einer Ultraschallvorrichtung 3. Sowohl die Endoskopievorrichtung 2 als auch die Ultraschallvorrichtung 3 dienen zum Erzeugen eines Bildes eines Objektes in Form eines Gewebes 4.

Bei der Endoskopievorrichtung 2 handelt es sich um ein starres Endoskop, das einen starren Endoskopschaft 21 aufweist, in dem sich eine Endoskopoptik zum Abbilden eines Objektes in eine Bildebene einer Kamera 22 der Endoskopievorrichtung 2 befindet. Eine mögliche Ausgestaltung der Endoskopievorrichtung 2 ist in der bereits erwähnten deutschen Patentanmeldung DE 10 2010 042 540.0 beschrieben. Die Kamera 22 und die Ultraschallvorrichtung 3 sind mit einem Datenverarbeitungssystem in Form eines Rechners 100 zum Aufbereiten (z.B. Digitalisieren und Filtern) und Darstellen der jeweils erzeugten Bilddaten verbunden.

An der Endoskopievorrichtung 2 sind Markierungselemente in Form von Markierungskugeln 51 - 53 angeordnet. Analog sind auch an einem Ultraschall köpf 31 der Ultraschallvorrichtung 3 Markierungskugeln 54 - 56 angeordnet, wobei die Markierungskugeln 51-56 eine Bestimmung der Position, d. h. sowohl des Ortes, an dem sich die jeweilige Vorrichtung befindet, als auch der Ausrichtung der jeweiligen Vorrichtung, ermöglichen. Hierzu weist die erfindungsgemäße Vorrichtung 1 eine Positionsbestimmungseinrichtung in Form eines klinischen Navigationssystems 6 auf, das eine Messkamera in Form einer Stereokamera 61 umfasst. Mit Hilfe der Stereokamera 61 kann die Position der an der Endoskopievorrichtung 2 angebrachten Markierungskugeln 51 - 53 und der an dem Ultraschallkopf 31 der Ultraschallvorrichtung 3 angeordneten Markierungskugeln 54 - 56 und damit der Endoskopievorrichtung 2 und der Ultraschallvorrichtung 3 bestimmt werden.

Insbesondere ist durch die Bestimmung der Position der Endoskopievorrichtung 2 auch die Position der Bildebene der Endoskopiekamera 22 sowie die Lage des vom Ultraschallkopf 31 erfassten Bereichs (z.B. eine Ebene) des Gewebes 4 bekannt und wird z.B. in Echtzeit bei einer (manuellen) Bewegung der Endoskopievorrichtung 2 und/oder des Ultraschallkopfes 31 aktualisiert. Nach einer Kalibrierung der Endoskopievorrichtung 2 und der Ultraschallvorrichtung 3 ist es daher möglich, das mit der Ultraschallvorrichtung 3 erzeugte Ultraschallbild perspektivisch korrekt mit dem von der Endoskopievorrichtung 2 erzeugten Endoskopiebild zu kombinieren. Hierzu werden (ersten) Koordinaten von Bildpunkten des Ultraschallbildes räumliche Koordinaten in einem der Positionsbestimmungsvorrichtung 6 (der Messkammer 61) zugeordneten räumlichen Koordinatensystem zugeordnet, wobei die den Ultraschallbildpunkten zuzuordnenden räumlichen Koordinaten von Punkten des mit der Ultraschallvorrichtung 3 erfassten Bereichs des Gewebes 4 sind.

Diese den Bildpunkten des Ultraschallbildes zugeordneten räumlichen Koordinaten werden unter Verwendung einer (simulierten) Abbildung, die durch das Kalibrieren der Endoskopievorrichtung 2 ermittelt wurde, in die (virtuelle) Bildebene der Kamera 22, d. h. in eine Ebene, die bezgl. ihrer Lage der Bildebene der Kamera 22 entspricht, abgebildet und (zweite) Koordinaten der Ultraschallbildpunkte bezüglich der Bildebene der Kamera 22 bestimmt. Durch Ersetzen und/oder Überlagern von Bildpunkten des mit der Endoskopievorrichtung 2 erzeugten Endoskopiebildes durch Bildpunkte des Ultraschallbildes, die entsprechende (zweite) Koordinaten (d.h. Koordinaten bzgl. der Bildebene der Kamera 22) aufweisen, wird ein kombiniertes Bild des Gewebes 4 erzeugt.

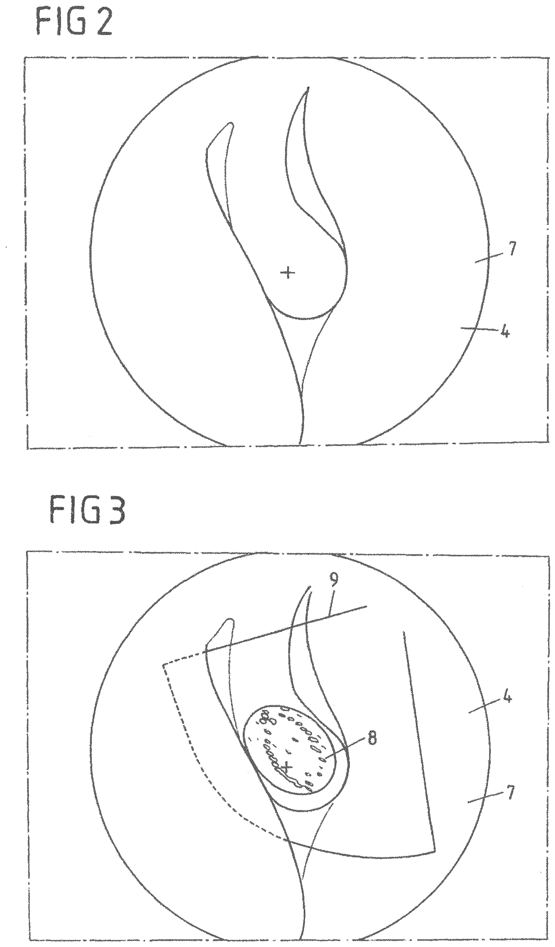

Ein Beispiel eines solchen perspektivisch korrekt kombinierten Bildes zeigt die Figur 3, wobei der Figur 3 ein mit der Endoskopievorrichtung 2 aufgenommenes Endoskopiebild zugrunde liegt, das in Figur 2 gezeigt ist.

Gemäß Figur 3 ist ein Teilbereich des Endoskopiebildes 7 durch ein Ultraschallbild 8 er-10 setzt, wobei die Koordinaten der Punkte des ursprünglichen Ultraschallbildes nach dem oben beschriebenen Verfahren transformiert wurden. Die auf diese Weise ermöglichte lagerichtige Integration des Ultraschallbildes in das Endoskopiebild stellt ergänzend zu der optischen Information des Endoskopiebildes eine Tiefeninformation des Gewebes zur Verfügung. Die Tiefe des Ultraschallbildes, d. h. Lage der Ebene, die durch den Ultraschallkopf erfasst und in dem Ultraschallbild 8 dargestellt wird, kann z. B. durch ein manuelles Bewegen des Ultraschallkopfes 31 der Ultraschallvorrichtung 3 verändert werden. Denkbar ist auch, dass anstelle des gezeigten 2D-Ultraschallbildes eine SD-Rekonstruktion des Gewebes integriert wird.

Wie oben erläutert, ist es möglich, dass ein Teilbereich des Gewebe 4 ausgewählt wird und das Endoskopiebild 7 nur in einem Abschnitt durch das Ultraschallbild 8 ersetzt oder überlagert wird, der dem ausgewählten Teilbereich des Gewebes 4 entspricht. Die Auswahl eines interessierenden Teilbereichs kann z.B. mit Hilfe einer Abstandsbestimmungsvorrichtung der Endoskopievorrichtung erfolgen, wobei mit einem Laser der Abstandsbestimmungsvorrichtung Lichtflecke auf dem Gewebe 4 erzeugt werden können, die den Teilbereich markieren. In den Figuren 2 und 3 ist die Position des Lichtflecks mit .einem Kreuz gekennzeichnet. Beispielsweise erfolgt das Markieren mit Hilfe des Lasers dadurch, dass mit dem Laser mehrere Stellen des Gewebes abgetastet und registriert werden, wie oben bereits erläutert.

In dem kombinierten Bild können auch zusätzliche Informationen eingeblendet werden, z. B. eine Information bezüglich der Ausrichtung des Ultraschallkopfes 31. Wie in Figur 3 gezeigt, kann dies z. B. durch .Einblenden einer Struktur 9, die die Lage des von dem Ultraschallkopf 31 erfassten Schnittkegels 9 kennzeichnet, erfolgen. Die. Struktur 9 ist eine "Augmented Reality"- Einblendung. Denkbar sind weitere derartige Einblendungen, die z.B. eine Richtung angeben, entlang der der Ultraschallkopf bewegt werden muss, um einen markierten Teilbereich des Gewebes zu erfassen.

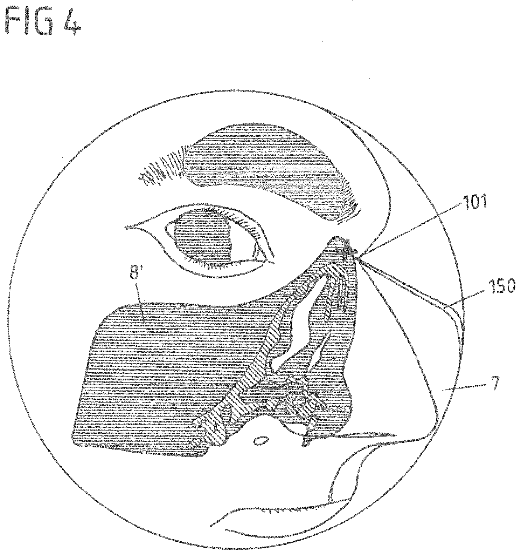

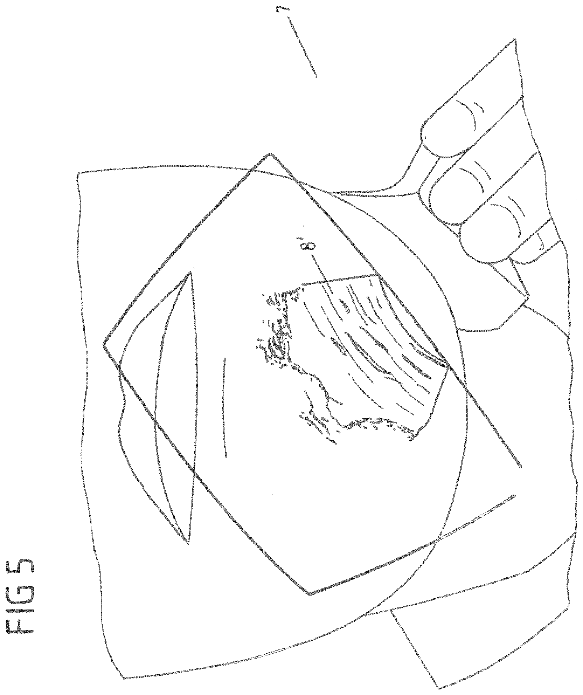

Die Figuren 4 und 5 zeigen jeweils ein kombiniertes Bild mit einem in ein erstes Bild 7 (z.B. per Endoskop oder einer sonstigen Kamera aufgenommen) eingeblendeten präoperativen CT-Bild 8'.

Des Weiteren ist in Fig. 4 ein navigiertes Instrument 150 dargestellt, d.h. ein Instrument, dessen räumliche Position per Navigationssystem (s.o.) bestimmt wurde, wobei das Instrument 150 insbesondere entsprechende Navigations-Markierungselemente aufweist. Die Position des Instrumentes (Werkzeug) 150 ist in dem kombinierten Bild lagerichtig in Bezug auf das CT-Bild 8' durch eine linienartige Markierung 101 gekennzeichnet. Darüber hinaus ist in Bezug auf CT-Bild 8' eine Verlängerung der Achse des Instrumentes 150 gekennzeichnet (Kreuz).

### Bezugszeichenliste

- 1: Vorrichtung
- 2: Endoskopievorrichtung
- 3: Ultraschallkopf
- 4: Gewebe
- 6: klinisches Navigationssystem
- 7: Endoskopiebild
- 8: Ultraschallbild
- 8': präoperatives CT-Bild
- 9: Struktur
- 21: Endoskopschaft
- 22: Kamera
- 31: Ultraschallkopf
- 61: Stereokamera
- 51-56: Markierungskugel
- 100: Rechner
- 101: Markierung
- 150: Instrument

## Patentansprüche

1. Auswahlverfahren zur Selektion von Bildpunkten bei der Darstellung eines Objektes bei intraoperativer Bildgebung, insbesondere eines biologischen Gewebes, mit den Schritten:
a) Erzeugen eines ersten Bildes (7) zumindest eines Teilbereichs des Objekts (4) mit Hilfe einer Endoskopievorrichtung als erster Vorrichtung (2);
b) Erzeugen eines zweiten Bildes (8, 8') zumindest des Teilbereichs des Objekts (4) aus Schritt a) mit Hilfe einer zweiten Vorrichtung (3);
c) Ermitteln von ersten Koordinaten zumindest einiger Bildpunkte des zweiten Bildes in einem ersten Koordinatensystem;
d) Ermitteln von zweiten Koordinaten der Bildpunkte des zweiten Bildes durch Abbilden der ersten Koordinaten in ein zweites Koordinatensystem, das von dem ersten Koordinatensystem verschieden und der ersten Vorrichtung (2) zugeordnet ist; und
e) Erzeugen eines kombinierten Bildes des Objekts (4) aus dem ersten Bild und für ausgewählte Bildpunkte aus dem zweiten Bild unter Verwendung der ermittelten zweiten Koordinaten der Bildpunkte des zweiten Bildes, **dadurch gekennzeichnet, dass**
f) die Selektion der zu kombinierenden Bildpunkte bestimmt wird durch die Auswahl eines Teilbereiches des Objektes (4), wobei
• die erste Vorrichtung (2) eine Lichtquelle aufweist und der auszuwählende Teilbereich des Objektes (4) durch Erzeugen mindestens eines Lichtflecks auf dem Objekt (4) mit Hilfe der Lichtquelle markiert wird, oder
• der Teilbereich des Objektes (4) mit Hilfe eines separaten, navigierten Instruments markiert wird,
g) so dass das Erzeugen des kombinierten Bildes bei der intraoperativen Bildgebung durch Ersetzen oder Überlagern von Bildpunkten des ersten Bildes (7) innerhalb des ausgewählten Teilbereichs des Objektes (4) durch die ausgewählten Bildpunkte des zweiten Bildes erfolgt.

2. Verfahren nach Anspruch 1, wobei die Markierung eines Teilbereiches des Objektes (4) mittels Lichtflecken **gekennzeichnet ist durch**
- Markieren eines räumlichen Teilbereichs des Objektes (4) mittels einer Mehrzahl von Lichtflecken;
- Ermitteln der räumlichen Position der ersten Vorrichtung (2) unter Verwendung einer Positionsbestimmungsvorrichtung (6);
- Ermitteln des Abstandes zwischen der ersten Vorrichtung (2) und den erzeugten Lichtflecken mit Hilfe einer Abstandsbestimmungsvorrichtung;
- wobei das Auswählen des Teilbereichs durch Ermitteln der räumlichen Position des Teilbereichs durch Bestimmen der räumlichen Koordinaten der Lichtflecke im Koordinatensystem der Positionsbestimmungsvorrichtung (6) unter Verwendung der ermittelten räumlichen Position der ersten Vorrichtung (2) und des ermittelten Abstandes erfolgt.

3. Verfahren nach einem der Ansprüche 1 oder 2, **dadurch gekennzeichnet, dass** der markierte räumliche Teilbereich zu einem geschlossenen Teilbereich extrapoliert wird und ein kombiniertes Bild nur erzeugt wird, wenn der mit der zweiten Vorrichtung (3) erfasste Bereich des Objektes (4) den geschlossenen Teilbereich schneidet.

4. Verfahren nach einem der vorhergehenden Ansprüche, **dadurch gekennzeichnet, dass** der geschlossene, extrapolierte Teilbereich, die räumlichen Koordinaten der Lichtflecke, die Teilbereiche aus Anspruch 1 und/oder zumindest einige der ermittelten Abstände in dem ersten und/oder dem zweiten Bild angezeigt wird.

5. Verfahren nach einem der Ansprüche 2 bis 4, **dadurch gekennzeichnet, dass** die Abstandsbestimmungsvorrichtung mit der ersten Vorrichtung (2) verbunden ist.

6. Verfahren nach einem der vorhergehenden Ansprüche, **dadurch gekennzeichnet, dass** die Selektion der zu kombinierenden Bildpunkte zusätzlich durch Anwenden einer booleschen Prädikatfunktion auf Bildpunkte des zweiten Bildes und/oder eines Bildanalyseverfahrens auf das zweite Bild eingeschränkt werden kann.

7. Verfahren nach Anspruch 1, **dadurch gekennzeichnet, dass** der Teilbereich des Objektes (4) unter Verwendung einer Rekonstruktion eines dreidimensionalen Bereichs des Objektes (4) ausgewählt wird, wobei die Rekonstruktion unter Verwendung einer Mehrzahl von mit der zweiten Vorrichtung (3) erzeugten Bildern generiert wird.

8. Verfahren nach Anspruch 7, **dadurch gekennzeichnet, dass** die Rekonstruktion des dreidimensionalen Bereichs des Objektes (4) eine Interpolation zwischen zwei oder mehr zeitlich aufeinanderfolgenden Bildern der zweiten Vorrichtung (3) in Abhängigkeit von ihrer jeweiligen, mittels der Positionsbestimmungsvorrichtung (6) ermittelten räumlichen Positionen umfasst.

9. Verfahren nach einem der vorhergehenden Ansprüche, **dadurch gekennzeichnet, dass** der Teilbereich des Objektes (4) unter Verwendung eines Ray-Casting-Verfahrens in dem ersten und/oder zweiten Bild (7) dargestellt wird, um einen Tiefeneindruck zu vermitteln.

10. Verfahren nach Anspruch 9, **dadurch gekennzeichnet, dass** das Ray-Casting-Verfahren eine Maximum-Intensity-Projektion, eine Average-Intensity-Projektion, eine frei definierbare Transferfunktion und/oder eine Oberflächen-Projektion verwendet.

11. Verfahren nach einem der vorhergehenden Ansprüche, **dadurch gekennzeichnet, dass** zu einem ersten Zeitpunkt Ausgangskoordinaten des Teilbereichs und zu einem zweiten Zeitpunkt aktualisierte Koordinaten des Teilbereichs in Abhängigkeit von der aktuellen Position und/oder Form des Objektes (4) bestimmt werden.

12. Verfahren nach einem der vorhergehenden Ansprüche, **dadurch gekennzeichnet, dass** das erste Bild (7) ein mit einer projektiven Optik auf einer 2D-Ebene erzeugtes Bild, insbesondere ein Endoskopiebild oder ein Stereoendoskopiebild ist.

13. Verfahren nach einem der vorhergehenden Ansprüche, **dadurch gekennzeichnet, dass** das zweite Bild ein intraoperatives Bild, insbesondere ein Ultraschallbild, oder ein präoperatives Bild, insbesondere ein MRT-Bild, ein Röntgenbild, ein nuklear-radiologisches Bild, ein Einzelphotonen-Emissions-Tomografie-Bild und/oder eine Fusion dieser Bilder und die zweite Vorrichtung (3) entsprechend eine Ultraschallvorrichtung, eine MRT-Vorrichtung, eine Röntgenvorrichtung, eine nuklear-radiologische Vorrichtung, eine MRT-Vorrichtung, eine Einzelphotonen-Emissions-Tomografie-Vorrichtung und/oder eine Bildfusionsvorrichtung ist.

14. Vorrichtung zur Darstellung eines Objektes zum Durchführen eines Verfahrens gemäß einem der vorhergehenden Ansprüche bei der intraoperativen Bildgebung, mit
- einer Endoskopievorrichtung als erster Vorrichtung (2) zum Erzeugen eines ersten Bildes (7) zumindest eines Teilbereichs eines Objekts (4);
- einer zweiten Vorrichtung (3) zum Erzeugen zumindest des Teilbereichs eines zweiten Bildes des Objekts (4) aus Schritt a);
- einer ersten Koordinatenermittlungseinrichtung zum Ermitteln von ersten Koordinaten zumindest einiger Bildpunkte des zweiten Bildes in einem ersten Koordinatensystem;
- einer zweiten Koordinatenermittlungseinrichtung zum Ermitteln von zweiten Koordinaten der Bildpunkte des zweiten Bildes durch Abbilden der ersten Koordinaten in ein zweites Koordinatensystem, das von dem ersten Koordinatensystem verschieden und der zweiten Vorrichtung (21) zugeordnet ist; und
- einer Bilderzeugungseinrichtung zum Erzeugen eines kombinierten Bildes des Objekts bei der intraoperativen Bildgebung aus dem ersten und dem zweiten Bild unter Verwendung der ermittelten zweiten Koordinaten der Bildpunkte des zweiten Bildes, wobei die erste Vorrichtung (2) eine Lichtquelle zum Erzeugen mindestens eines Lichtflecks auf dem Objekt (4) aufweist oder ein separates, navigiertes Instrument zur Auswahl des Teilbereichs des Objekts (4) vorhanden ist.

15. Vorrichtung nach Anspruch 14, **gekennzeichnet durch** an der ersten und/oder der zweiten Vorrichtung (2, 3) angeordnete Markierungselemente (51-56) sowie eine Positionsbestimmungsvorrichtung (6) zur Bestimmung der räumlichen Position der Markierungselemente (51-56) und somit der räumlichen Position der ersten und/oder der zweiten Vorrichtung (2, 3).

## Claims

1. A method for selection of image points in the representation of an object in intraoperative imaging, in particular a biological tissue, comprising the steps of:
a) Generating a first image (7) of at least a sub-region of the object (4) by means of an endoscopy device as a first device (2);
b) Generating a second image (8, 8') of at least the sub-region of the object (4) from step a) by means of a second device (3);
c) Ascertaining first coordinates of at least some image points of the second image in a first coordinate system;
d) Asceratining second coordinates of the image points of the second image by mapping the first coordinates into a second coordinate system, which is different from the first coordinate system and is assigned to the first device (2); and
e) Generating a combined image of the object (4) from the first image and for selected image points from the second image using the determined second coordinates of the image points of the second image, **characterized in that**
f) the selection of image points to be combined is determined by the selection of a subarea of the object (4), wherein
• the first device (2) comprises a light source and the subarea of the object (4) to be selected is marked by generating at least one light spot on the object (4) with aid of the light source, or
• the subarea of the object (4) is marked with a separate, navigated instrument,
g) such that generating of the combined image in the intraoperative imaging occurs by replacing or superimposing image points of the first image (7) within the selected subarea of the object (4) through the selected image points of the second image.

2. The method according to claim 1, wherein the marking of a subarea of the object (4) with light spots is **characterized by**
- Marking a spatial subarea of the object (4) with a plurality of light spots;
- Determining a spatial position of the first device (2) using a position determining device (6);
- Determining the distance between the first device (2) and the generated light spots with aid of a distance determining device;
- wherein the selection of the subarea occurs by determining the spatial position of the subarea by determining the spatial coordinates of the light spots in the coordinate system of the position determining device (6) using the determined spatial position of the first device (2) and the determined distance.

3. The method according to any one of claims 1 or 2, **characterized in that** the marked spatial subarea is extrapolated to a closed subarea, and a combined image is generated only when the area of the object (4) detected by the second device (3) intersects the closed subarea.

4. The method according to any one of the preceding claims, **characterized in that** the closed, extrapolated subarea, the spatial coordinates of the light spots, the subareas of claim 1 and/or at least some of the determined distances are displayed in the first and/or the second image.

5. The method according to any one of claims 2 to 4, **characterized in that** the distance determining device is connected to the first device (2).

6. The method according to any one of the preceding claims, **characterized in that** the selection of the image points to be combined can additionally be limited by applying a Boolean predicate function to image points of the second image and/or an image analysis method to the second image.

7. The method according to claim 1, **characterized in that** the subarea of the object (4) is selected using a reconstruction of a three-dimensional area of the object (4), wherein the reconstruction is generated using a plurality of images generated with the second device (3).

8. The method according to claim 7, **characterized in that** the reconstruction of the three-dimensional area of the object (4) comprises an interpolation between two or more images of the second device (3) in chronological succession as a function of their respective spatial positions determined with the position determining device (6).

9. The method according to any one of the preceding claims, **characterized in that** the subarea of the object (4) is represented using a Ray Casting method in the first and/or the second image (7) in order to impart a depth impression.

10. The method according to claim 9, **characterized in that** the ray casting method uses a maximum intensity projection, an average intensity projection, a freely definable transfer function and/or a surface projection.

11. The method according to any one of the preceding claims, **characterized in that** at a first point in time, initial coordinates of the subarea and at a second point in time, updated coordinates of the subarea are determined as a function of the current position and/or form of the object (4).

12. The method according to any one of the preceding claims, **characterized in that** the first image (7) is an image generated with projective optics on a 2D plane, in particular an endoscopic image or a stereoendoscopic image.

13. The method according to any one of the preceding claims, **characterized in that** the second image is an intraoperative image, in particular an ultrasound image, or a preoperative image, in particular an MRI image, an X-ray image, a nuclear radiological image, a single-photon emission tomography image and/or a fusion of these images and the second device (3) is accordingly an ultrasound device, an MRI device, an X-ray device, a nuclear radiological device, a single-photon emission tomography device and/or an image fusion device.

14. A device for the representation of an object for performing a method in intraoperative imaging according to any one of the preceding claims with
- an endoscopy device as first device (2) for generating a first image (7) of at least a subarea of the object (4);
- a second device (3) for generating at least the subarea of a second image of the object (4) from step a);
- a first coordinate determination device for determining first coordinates of at least some image points of the second image in a first coordinate system;
- a second coordinate determination device for determining second coordinates of image points of the second image by mapping the first coordinates into a second coordinate system, which is different from the first coordinate system and is assigned to the second device (3); and
- an image generating device for generating a combined image of the object in intraoperative imaging from the first and the second image using the determined second coordinates of the image points of the second image, wherein the first device (2) comprises a light source for generating at least one light spot on the object (4) or a separate, navigated instrument for selecting the subarea of the object (4).

15. The device according to claim 14, **characterized by** marking elements (51-56) arranged on the first and/or the second device (2, 3), as well as a position determining device (6) for determining the spatial position of the marking elements (51-56) and thus the spatial position of the first and/or the second device (2, 3).

## Revendications

1. Méthode de sélection de points d'image dans la représentation d'un objet dans l'imagerie intra-opératoire, en particulier un tissue biologique, comprenant les étapes:
a) Générer une première image (7) d'au moins une région partielle de l'objet (4) à l'aide d'un appareil d'endoscopie comme premier appareil (2);
b) Générer une seconde image (8, 8') au moins de la région partielle de l'objet (4) de l'étape a) à l'aide d'un second appareil (3);
c) Déterminer des premières coordonnées d'au moins certains points d'image de la seconde image dans un premier système de coordonnées;
d) Déterminer des secondes coordonnées des points d'image de la seconde image en mappant les premières coordonnées dans un second système de coordonnées qui est différent du premier système de coordonnées et qui est attribué au premier appareil (2); et
e) Générer une image combinée de l'objet (4) à partir de la première image et pour des points d'image sélectionnés à partir de la seconde image en utilisant les secondes coordonnées des points d'image de la seconde image qui ont été déterminées, **caractérisé en ce que**
f) la sélection des points d'image à combiner est déterminée par la sélection d'une région partielle de l'objet (4), dans lequel
• le premier appareil (2) comprend une source de lumière et la région partielle de l'objet (4) à sélectionner est marquée en générant au moins un point lumineux sur l'objet (4) à l'aide de la source de lumière, ou
• la région partielle de l'objet (4) est marquée à l'aide d'un instrument navigué séparé,
g) de sorte que la génération de l'image combinée dans l'imagerie intra-opératoire se produit en remplaçant ou superposant des points d'image de la première image (7) dans la région partielle de l'objet (4) par les points d'image sélectionnés de la seconde image.

2. Méthode selon la revendication 1, dans laquelle le marquage d'une région partielle de l'objet (4) à l'aide de points lumineux est **caractérisé par**
- marquer une région partielle spatiale de l'objet (4) à l'aide d'une pluralité de points lumineux;
- déterminer la position spatiale du premier appareil (2) en utilisant un appareil de détermination de position (6);
- déterminer la distance entre le premier appareil (2) et les points lumineux générés avec l'aide d'un appareil de détermination de distance;
- dans laquelle la sélection de la région partielle se produit en déterminant la position spatiale de la région partielle en déterminant les coordonnées spatiales des points lumineux dans le système de coordonnées de l'appareil de détermination de position (6) en utilisant la position spatiale déterminé du premier appareil (2) et de la distance déterminée.

3. Méthode selon une des revendications 1 ou 2, **caractérisée en ce que** la région partielle spatiale marquée est extrapolée à une région partielle fermée, et une image combinée est générée seulement si la région de l'objet (4) détectée par le seconde appareil (3) coupe la région partielle fermée.

4. Méthode selon l'une quelconque des revendications précédentes, **caractérisée en ce que** la région partielle extrapolée fermée, les coordonnée spatiales des points lumineux, les régions partielles de la revendication 1 et/ou au moins certaines des distances déterminées sont affichées dans la première et/ou la seconde image.

5. Méthode selon l'une quelconque des revendications 2 à 4, **caractérisée en ce que** l'appareil de détermination de distance est relié au premier appareil (2).

6. Méthode selon l'une des revendications précédentes, **caractérisée en ce que** la sélection des points d'images à combiner peut en outre être limitée en appliquant une fonction de prédicat booléen à des points d'image du seconde image et/ou un procédé d'analyse d'image à la seconde image.

7. Méthode selon la revendication 1, **caractérisée en ce que** la région partielle de l'objet (4) est sélectionnée en utilisant une reconstruction d'une région tridimensionelle de l'objet (4), dans laquelle la reconstruction est générée en utilisant une pluralité d'images générées avec le second appareil (3).

8. Méthode selon la revendication 7, **caractérisée en ce que** la reconstruction de la région tridimensionelle de l'objet (4) comprend une interpolation entre deux ou plusieurs images du second appareil (3) en succession chronologique en fonction de leurs positions spatiales respectives déterminées à l'aide de l'appareil de détermination de position (6).

9. Méthode selon l'une quelconque des revendications précédentes, **caractérisée en ce que** la région partielle de l'objet (4) est représenté en utilisant une méthode raycasting dans la première et/ou la seconde image (7), afin de procurer une impression de profondeur.

10. Méthode selon la revendication 9, **caractérisée en ce que** la méthode raycasting utilise une projection d'intensité maximale, une projection d'intensité moyenne, une fonction de transfert librement définissable et/ou une projection de surface.

11. Méthode selon l'une quelconque des revendications précédentes, **caractérisé en ce que**, dans un premier instant, des coordonnées initiales de la région partielle et dans un seconde instant des coordonnées actualisées de la région partielle sont déterminées en fonction de la position actuelle et/ou de la forme de l'objet (4).

12. Méthode selon l'une quelconque des revendications précédentes, **caractérisé en ce que** la première image (7) est une image générée avec une optique projective sur un plan 2D, en particulier une image endoscopique ou une image stéréoendoscopique.

13. Méthode selon l'une quelconque des revendications précédentes, **caractérisée en ce que** la seconde image est une image intra-opératoire, en particulier une image ultrasonore, ou une image préopératoire, en particulier une image IRM, une image radiographique, une image nucléaire-radiologique, une image de tomographie par émission de photons unique et/ou une fusion de ces images et le second appareil (3) est adéquatement un appareil à ultrasons, un appareil IRM, un appareil à rayon X, un appareil radiologique nucléaire, un appareil de tomographie par émission de photons unique et/ou un appareil de fusion d'image.

14. Appareil pour la représentation d'un objet pour effectuer une méthode en imagerie intra-opératoire selon l'une quelconque des revendications précédentes avec
- un appareil d'endoscopie comme premier appareil (2) pour générer une première image (7) d'au moins une région partielle d'un objet (4);
- un seconde appareil (3) pour générer au moins la région partielle d'une seconde image de l'objet (4) de l'étape a);
- un premier appareil de détermination de coordonnées pour déterminer des premières coordonnées d'au moins certaines points d'images de la seconde image dans un premier système de coordonnées;
- un seconde appareil de détermination de coordonnées pour déterminer des secondes coordonnées des points d'image de la seconde image en mappant les premières coordonnées dans un seconde système de coordonnées qui est différent du premier système de coordonnées et qui est attribué au seconde appareil (3); et
- un appareil de génération d'image pour générer une image combinée de l'objet dans l'imagerie intra-opératoire à partir de la première et la seconde image en utilisant les secondes coordonnées des points d'images de la seconde image qui ont été déterminées, dans laquelle le premier appareil (2) comprend une source de lumière pour générer au moins un point lumineux sur l'objet (4) ou un instrument navigué séparé pour sélectionner la région partielle de l'objet (4).

15. Appareil selon la revendication 14, **caractérisé par** des éléments de marquage (51-56) agencés sur le premier et/ou le seconde appareil (2, 3), aussi bien qu'un appareil de détermination de position (6) pour déterminer la position spatiale des éléments de marquage (51-56) et ainsi la position spatiale du premier et/ou du seconde appareil (2, 3).
